# EUROPEAN PATENT SPECIFICATION

(11) **EP 4 289 466 B1**
(45) Date of publication and mention of the grant of the patent: **06.05.2026**
(21) Application number: 23184845.8
(22) Date of filing: 31.05.2019
(51) Int. Cl.: A61M 16/04, A61F 2/20, A61M 5/32, A61M 16/00, A61M 29/00, A61B 5/06, A61B 5/107, A61B 5/00

(54) **SYSTEMS, APPARATUS, AND METHODS FOR PERFORMING A PERCUTANEOUS TRACHEOSTOMY**
SYSTEME, VORRICHTUNG UND VERFAHREN ZUR DURCHFÜHRUNG EINER PERKUTANEN TRACHEOSTOMIE
SYSTÈMES, APPAREILS ET PROCÉDÉS POUR RÉALISER UNE TRACHÉOSTOMIE PERCUTANÉE

(30) Priority: 01.06.2018 US 201862679282 P; 07.09.2018 US 201862728450 P
(43) Date of publication of application: 13.12.2023
(62) Divisional of application: 19811977.8
(73) Proprietor: CoapTech, Inc., Baltimore MD Maryland 21230 (US)
(72) Inventor: TROPELLO, Steven P., Baltimore, 21212 (US); CAROLAN, Howard, Baltimore, 21224 (US); GOLDWASSER, Elisabeth, Baltimore, 21224 (US)
(74) Representative: Cooley (UK) LLP

(56) References cited:
- WO-A1-2010/129327
- WO-A1-2017/216650
- IT-A1- MI20 111 820

## Description

### Background

Embodiments described herein relate to a system of claim 1.

Some patients suffer from medical conditions that impair the patient's ability to breath. In such patients, a tracheostomy procedure may be beneficial. For some patients, percutaneous tracheostomy is a preferable procedure over open surgical tracheostomy because it is safer and less expensive. Some percutaneous tracheostomy techniques include the Ciaglia technique (serial or one-step dilation), the Griggs technique (wire forceps), and a translaryngeal tracheostomy (or Fantoni) technique. Generally, a percutaneous tracheostomy procedure requires safely puncturing the anterior trachea of a patient and inserting a guidewire using the Seldinger technique. Typically, the entry site used for the puncture and subsequent guidewire insertion is between the first and second or second and third tracheal rings.

Identifying the ideal site for tracheal puncture, however, can be challenging. Advanced tools such as bronchoscopy and ultrasound have been used to improve upon physical exam landmarks. Bronchoscopes, like many advanced procedural tools, can be expensive, have issues with sterility, can malfunction, and often lack immediate availability. While ultrasound, unlike bronchoscopy, can identify tracheal rings, thyroid isthmus and proximal blood vessels to enable a safer needle insertion, ultrasound alone lacks the ability to visualize the posterior trachea and thus the patient can be at risk for posterior trachea wall damage. Without bronchoscopy, the damaged posterior wall cannot be accessed during dilatational percutaneous tracheostomy. Furthermore, standard bronchoscopes often inhibit adequate ventilation to the patient during a percutaneous tracheostomy procedure, raising additional risks for the patient.

Thus, there is a need for systems, apparatus, and methods of performing a percutaneous tracheostomy which reduce risks to the patient and allows for the percutaneous tracheostomy to be quickly and easily performed.

WO2017/216650 discloses an endotracheal tube for protecting the trachea and oesophagus from injury during the percutaneous dilatational tracheostomy procedure and bronchoscopy. The ET tube is fabricated with and without non-penetrable material piece. The ET tube has upper and lower portions. The upper portion of the ET tube is similar to cuffed endotracheal tubes and leads to a lower portion through proximal bevel. A short, thin cuff separates an upper portion from the lower portion. The lower portion is U-shaped, which provides bronchoscopic visualization of the anterior wall of trachea. The non-penetrable material covers the inner surface of the ET tube with non-penetrable material piece. This layer prevents the damage to respiratory canal during PDT procedure. A distal bevel of the ET facilitates induction of the ET tube into respiratory canal. Two radiopaque lines on the mid lateral sides of the tracheal tube help for locating right position of the tube.

### Summary

In accordance with one aspect of the invention, there is provided a system for performing a percutaneous tracheostomy as defined by claim 1. Optional features are defined by the dependent claims.

### Brief Description of the Drawings

FIG. 1 is a schematic illustration of a guidewire placement system, according to an embodiment.
FIGS. 2A and 2B are schematic illustrations of a front view and a cross-sectional side view, respectively, of a portion of human anatomy.
FIG. 2C is a schematic illustration of a cross-sectional side view of a portion of human anatomy engaged with a portion of the guidewire placement system of FIG. 1, according to an embodiment.
FIGS. 3A-3M are schematic illustrations of a guidewire placement system in various stages of operation, according to an embodiment.
FIG. 4 is a schematic illustration of a guidewire placement system, according to an embodiment.
FIG. 5 is a schematic illustration of a guidewire placement system, according to an embodiment.
FIG. 6 is a schematic illustration of a guidewire placement system, according to an embodiment.
FIG. 7 is a flow chart of a method, according to an embodiment.

### Detailed Description

It is disclosed but not claimed that a system includes an inflation assembly and a guidewire assembly. The inflation assembly can include an elongated tube, an inflatable member, and a magnetic member. The elongated tube can have a first end, a second end, and can define a lumen. The inflatable member can be coupled to the first end of the elongated tube and can be fluidically coupled to the lumen such that the inflatable member can receive fluid via the lumen. The magnetic member can be coupled to the first end of the elongated tube such that movement of the magnetic member can cause corresponding movement of the first end of the elongated tube. The guidewire assembly can include a guidewire having a first end and a second end. The first end of the guidewire assembly can include a coupling member, the coupling member configured to couple to the inflatable member such that translation of the elongated tube translates the guidewire assembly.

It is disclosed but not claimed that a method can include translating a tubular member through an orifice of a patient, through a cricoid ring of the patient, and into an upper trachea of the patient. A first end of an elongated tube can be translated through a lumen of the tubular member such that an inflatable member and a magnetic member of the elongated tube extends from a first end of the tubular member and is disposed in the upper trachea of the patient. An external magnetic assembly can be disposed on an anterior neck of the patient such that the magnetic member of the elongated tube is urged toward the anterior neck of the patient and the inflatable member is disposed against an inner surface of the upper trachea. The inflatable member can then be inflated via a lumen of the elongated tube such that the inflatable member transitions from an uninflated configuration to an inflated configuration. A coupling member of a guidewire assembly can be translated through the anterior neck of the patient and into the upper trachea of the patient. The guidewire assembly can include a guidewire having a first end coupled to the coupling member and a second end disposed outside the patient, the guidewire extending through the anterior neck of the patient. The coupling member can be coupled to the inflatable member.

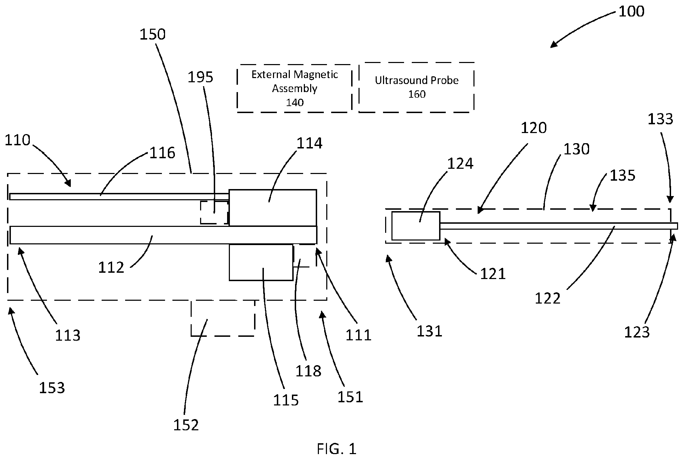

FIG. 1 is a schematic representation of a system 100. The system 100 includes an inflation assembly 110, a guidewire assembly 120, and a tubular member 150. The system 100 can optionally also include an external magnetic assembly 140 and an ultrasound probe 160. The inflation assembly 110 can include an elongated tube 112, an inflatable member 114, and a magnetic member 115. The inflation assembly 110 may optionally include a barrier member 195. The elongated tube 112 can have a first end 111 and a second end 113. In some embodiments, the elongated tube 112 can have a length sufficient to extend from at least an oral or nasal orifice of a patient to the trachea of the patient. The inflatable member 114 and the magnetic member 115 can be coupled to the elongated tube 112 at or near the first end 111 of the elongated tube 112. The inflation assembly 110 can include an inflation lumen 116 in fluid communication with the inflatable member 114. In some embodiments, the inflation lumen 116 can be disposed within and/or be defined by the elongated tube 112.

In some implementations, a light source 118 may be disposed on or near the first end 111 of the elongated tube 112. The light source 118 may produce sufficient light such that light can emit from the light source 118, through the tracheal wall, to the surface of the neck and be visible to a user (e.g., a clinician). Thus, the user may be able to determine the location of the first end 111 of the elongated tube 112 based, at least in part, on the location of light emitting through the patient's skin. In some embodiments, the light source 118 and the tubular member 150 may be configured such that light emitted by the light source 118 is partially or fully blocked by the tubular member 150 when the first end 111 of the elongated tube 112 is disposed within the tubular member 150 such that the light emitted by the light member is not visible on the skin of the patient or is more dim compared to when the first end 111 of the elongated tube 112 is not within the tubular member 150. Thus, when the elongated tube 112 is translated relative to the tubular member 150 such that the first end 111 extends from the tubular member 150 when the elongated tube 112 and the tubular member 150 are at least partially inserted into the patient, the light emitted from the light source 118 may become visible or more visible when the first end 111 is extended from an end of the tubular member 150. The light source 118 can be, for example, a light emitting diode (LED).

The magnetic member 115 can be any suitable magnetic member configured such that movement of the magnetic member 115 causes corresponding movement of the first end 111 of the elongated tube 112. The magnetic member 115 can have any suitable shape. For example, in some embodiments, the magnetic member 115 can be shaped as an elongated rectangle. In some embodiments, the magnetic member 115 can be shaped as a cylinder. In some embodiments, the magnetic member 115 may be arcuate. In some embodiments, the magnetic member 115 is coupled directly to the elongated tube 112. In some embodiments, the magnetic member 115 is disposed within the inflatable member 114 and at least partially surrounded by the inflatable member 114. In some embodiments, the magnetic member 115 is coupled directly to the inflatable member 114. In some embodiments, the system 100 includes two or more magnetic members 115.

In some embodiments, the inflatable member 114 can surround the elongated tube 112 in an inflated and/or uninflated configuration. In some embodiments, the inflatable member 114 can extend laterally from the elongated tube 112 in an inflated and/or uninflated configuration. In some embodiments, the inflatable member 114 can extend distally from the first end 111 of the elongated tube 112 in an inflated and/or uninflated configuration. In some embodiments, the inflatable member 114 can be disposed on the elongated tube 112 such that a portion of the elongated tube 112 extends distally of the inflatable member 114 when the inflatable member 114 is in an inflated and/or uninflated configuration. In some embodiments, the inflatable member 114 can have two ends (e.g., cuffs), and each end can be sealed to an outer surface of the elongated tube 112. The elongated tube 112 can define one or more inflation ports or holes such that the inflation lumen 116 can be in fluid communication with the interior of the inflatable member 114 for transitioning the inflatable member 114 between an uninflated and an inflated configuration. In some embodiments, the inflatable member 114 can be formed on or as a part of a rigid subassembly, and the rigid subassembly can receive the elongated tube 112 within an orifice of the subassembly and the elongated tube 112 can then be sealed to the subassembly.

In some embodiments, the inflatable member 114 can be formed in any suitable shape, in any suitable size, and of any suitable material. For example, the inflatable member 114 can be elliptical, spherical, cylindrical, rectangular, tear drop, or any other suitable shape. In some embodiments, the shape can be chosen based on the particular application of the system 100. For example, the shape of the inflatable member 114 may be selected to improve ultrasound visualization in particular regions of a patient's body. Furthermore, the inflatable member 114 can be sized for improved engagement and retention between the inflatable member 114 and the guidewire assembly 120.

The inflatable member 114 can be sufficiently pliable such that the inflatable member 114 (e.g., when inflated) can be punctured (e.g., by a needle) to define a pinhole in the wall of the inflatable member 114 rather than bursting or tearing as a result of puncture. In some embodiments, the inflatable member 114 can be formed of, for example, polyurethane, silicone, and/or polyvinyl chloride (PVC). In some embodiments, the inflatable member 114 can have any suitable material properties, wall thicknesses, and/ or inflated outermost diameters.

In some embodiments, for example, the inflatable member 114 can be elliptical in shape and formed of a low durometer urethane. The inflatable member 114 can have an outermost diameter ranging from about 40 mm to about 55 mm in an inflated configuration, and a length of about 55 mm. The inflatable member 114 can have a diameter at each end ranging from about 5.46 mm to about 5.72 mm. The wall thickness at the maximum balloon diameter in the inflated configuration can be between about 0.029 mm and about 0.038 mm. The inflatable member 114 can be filled with up to, for example, about 50 ml of fluid in the inflated configuration.

The guidewire assembly 120 can include a guidewire 122 having a first end 121 and a second end 123 and a coupling member 124 disposed at the first end 121 of the guidewire 122. The coupling member 124 can be configured to couple to the inflatable member 114 such that, when coupled, translation of the inflation assembly 110 (e.g., translation of the elongated tube 112 via pulling on the second end 113) can translate the guidewire assembly 120. For example, if the inflatable member 114 is moved in a first direction due to a force applied to the elongated tube 112, the coupling of the coupling member 124 to the inflatable member 114 can cause the coupling member 124 and the guidewire 122 to also move in the first direction. The coupling member 124 can be configured to couple with the inflatable member 114 via, for example, being captured by the inflatable member 114, caught within an interior region of the inflatable member, or engaged with a surface of the inflatable member 114.

In some embodiments, the coupling member 124 can be distinct from the guidewire 122 and fixedly coupled to the guidewire 122 (e.g., via adhesive). For example, in some embodiments, the coupling member 124 can include a first magnetic member configured to couple to a second magnetic member of the inflatable member 114.

In some embodiments, the guidewire 122 can include the coupling member 124. For example, the coupling member 124 can be monolithically formed with a shaft of the guidewire 122 such that the guidewire assembly 120 is a one piece structure. Similarly, in some embodiments, the coupling member 124 and the guidewire 122 can be formed of the same material or materials. In some embodiments, the coupling member 124 can be shaped such that the coupling member 124 can engage with at least one portion of a wall of the inflatable member 114. For example, the coupling member 124 can have a planar or a multiplanar shape and can be formed as a pigtail, hook, coil, or corkscrew-shaped end to the guidewire 122. Thus, in some embodiments, the first end 121 of the guidewire 122 can be retained within or near the inflatable member 122 by the coupling member 124 when the coupling member 124 is disposed within the inflatable member 114. In some embodiments, the coupling member 124 can be disposed outside of the inflatable member 114 with the guidewire 122 passing through a first wall portion and a second, oppositely disposed wall portion of the inflatable member 114 such that the guidewire 122 is retained by the inflatable member 114 due to the interaction between the coupling member 124 and the first wall portion of the inflatable member 114. In some embodiments, the coupling member 124 can be partially disposed within the inflatable member 114 and partially disposed outside of the inflatable member 114 such that the guidewire assembly 120 is coupled to the inflatable member 114 for translation of the guidewire assembly 120 via movement of the inflation assembly 110.

In some embodiments, the coupling member 124 can be configured to transition between a first configuration for insertion and a second configuration for retention or coupling. For example, the coupling member 124 can have a smaller lateral extent (e.g., outermost diameter) relative to a central axis of the guidewire 122 in the first configuration than in the second configuration such that the coupling member 124 can fit inside the lumen 135 of the needle 130 in the first configuration and can expand to retain the guidewire 122 relative to the inflatable member 114 in the second configuration. In some embodiments, the coupling member 124 can have a first shape in the first configuration and a second shape in the second configuration such that the coupling member 124 can travel through an opening in at least one sidewall of the inflatable member 114 in the first configuration and can engage a sidewall of the inflatable member 114 in the second configuration such that the coupling member 124 is retained by the inflatable member 114. In some embodiments, the coupling member 124 can be biased toward the second configuration such that, in the absence of external forces on the coupling member 124, the coupling member 124 will assume the second configuration. In some embodiments, in the first configuration the coupling member 124 can be elongated such that the coupling member is shaped as a straight wire. The second configuration can correspond to an unbiased shape or configuration of the coupling member (e.g., a pigtail, hook, coil, or corkscrew-shape). In some embodiments, the guidewire 122 and/or the coupling member 124 can be formed of a shape-memory material such as, for example, Nitinol.

In some embodiments, when the coupling member 124 is within the lumen 135 of the needle 130, the needle 130 can compress the coupling member 124 such that the coupling member is in the first configuration. Thus, the coupling member 124 can have a smaller lateral extent relative to a central axis of the guidewire 122 (e.g., outermost diameter) when disposed within the lumen 135 of the needle 130 than when not within the needle 130. In some embodiments, the lumen 135 and the coupling member 124 can be structured and sized such that the coupling member 124 can be straight or substantially straight within the lumen 135 of the needle 130. For example, the lumen 135 can have an inner diameter similar to an outer diameter of the coupling member 124 (e.g., an outer diameter of a wire forming the coupling member 124 portion of the guidewire assembly 120) such that the coupling member 124 can be laterally compressed to a shape with a smaller outer diameter and/or elongated within the lumen 135 of the needle 130. In some embodiments, the outer diameter of a wire forming the coupling member 124 and the inner diameter of the lumen 135 can be relatively sized such that the outer diameter of the wire forming the coupling member 124 is slightly smaller than the inner diameter of the lumen 135 and the coupling member 124 and the inner surface of the needle 130 defining the lumen 130 can have a slip fit engagement. Thus, when the coupling member 124 is threaded into the lumen 135 of the needle 130, the wire forming the coupling member 124 is straightened out to correspond to the shape of the lumen 135. As the coupling member 124 is translated out of the first end 131 of the needle 130, the coupling member 124 can transition from the first configuration to the second configuration. For example, as the coupling member 124 is extended from the first end 131 of the needle 130, the portion of the coupling member 124 extending from the first end 131 can transition toward the second configuration due to being biased toward the second configuration, while the portion of the coupling member 124 remaining within the lumen 135 of the needle 130 can remain in the first configuration. When the coupling member 124 is entirely outside of the needle 130, the coupling member 124 can be entirely in the second configuration.

In some embodiments, the coupling member 124 can be configured to be translated in a first direction by the inflatable member 114 if a translation force on the inflatable member 114 (e.g., a translating force on the inflatable member 114 and/or a force holding the inflatable member 114 stationary) is greater than a force in a direction opposite of the translation force on the coupling member 124. If the force on the coupling member 124 is opposite and greater than the translation force on the inflatable member 114, the coupling member 124 and the inflatable member 114 can be configured to decouple. For example, in some embodiments in which the coupling member 124 is a pigtail-shaped end to the guidewire 122, the application of sufficient force to the coupling member 124 in a direction opposite a force being applied to the inflatable member 114 can cause the pigtail-shaped end to straighten and decouple from the inflatable member 114. In some embodiments, the application of sufficient force to the coupling member 124 in a direction opposite a force being applied to the inflatable member 114 may cause the coupling member to tear a sidewall of the inflatable member such that the inflatable member 114 and the coupling member 124 are decoupled. Thus, in some embodiments the coupling member 124 and the inflatable member 114 can be decoupled via applying oppositely directing pulling forces to each of the coupling member 124 and the inflatable member 114. In some embodiments, the coupling member 124 and the inflatable member 114 can be engaged such that the release force (e.g., via oppositely directing pulling forces) necessary to separate the coupling member 124 from the inflatable member 114 is a force greater than the maximum force applied to the guidewire 122 (and therefore coupling member 124) in an opposite direction than the inflatable member 114 during withdrawal of the coupling member 124 from the patient via pulling on the inflation assembly 110. Thus, the release force is sufficiently high such that the inflatable member 114 and the coupling member 124 will not be separated during the withdrawal of the coupling member 124 of the guidewire 122 inadvertently during withdrawal, but can be separated via, for example, pulling by the user when the inflatable member 114 and the coupling member 124 are outside of the patient's body. For example, in some embodiments, the release force can be at least about 0.25 lbs of force, at least about 0.5 lbs of force, or at least about 1.5 lbs of force. In some applications of the system 100, the release force may be greater or smaller depending on the resistive forces the coupling member 124 and guidewire 122 may experience during withdrawal via a withdrawal force on the inflation assembly 110. In some embodiments, the coupling member 124 and the inflatable member 114 can be engaged such that the release force (e.g., via oppositely directing pulling forces) necessary to separate the coupling member 124 from the inflatable member 114 is a force less than the force applied to the guidewire 122 (and therefore coupling member 124) in a direction away from the patient (e.g., away from the patient's anterior neck) during withdrawal of the inflation assembly 110 from the patient via a patient orifice via pulling on the elongated tube 112. Thus, the release force can be sufficiently low such that the inflatable member 114 and the coupling member 124 will not be inadvertently separated during the movement of the coupling member 124 of the guidewire 122 via movement of the inflatable member 114 within, for example, the trachea of the patient, but can be separated via, for example, pulling by the user on the elongated tube 112 and the guidewire 122. In some embodiments, a user can decouple the inflatable member 114 from the coupling member 124 by pushing the inflatable member 114 along the coupling member 124 toward an end of the coupling member 124 such that the coupling member 124 is translated through the opening created by the needle 130 while in a straight or non-straight configuration.

In some embodiments, the coupling member 124 can be configured to pierce the inflatable member 114 such that the coupling member 124 can be inserted into and/or through the inflatable member 114. In some embodiments, the system 100 can optionally include a needle 130 having a first end 131, a second end 133, and defining a lumen 135. The first end 131 can have any suitable shape configured to pierce and create access to the inflatable member 114. For example, the first end 131 can have a sharpened tip that can be tapered. The lumen 135 can be sized such that the coupling member 124 of the guidewire assembly 120 can be translated through the second end 133, through the lumen 135, and through the first end 131 of the needle 130. In some embodiments, the needle 130 can be inserted through an anterior neck and trachea wall of the patient and through a sidewall of the inflatable member 114. The coupling member 124 and a portion of the guidewire 122 can then be translated through the lumen 135 of the needle 130 such that at least one of the coupling member 124 and a portion of the guidewire 122 is at least partially disposed within inflatable member 114. The needle 130 can then be removed from the inflatable member 114 via translating the needle 130 along the guidewire 122.

The optional barrier member 195 may be a portion of the 110 inflation assembly 110 that is more resistant to puncturing or tearing (e.g., by a needle) than the inflatable member 114 or a portion of the inflatable member 114. In some implementations, the barrier member 195 may be arranged in any suitable location relative to a portion of the inflatable member 114 that is intended to be pierced in which the barrier member can prevent a needle from passing through the inflatable member 114 and puncturing a posterior tracheal wall of the patient. In some implementations, the barrier member 195 may form a portion of a sidewall of the inflatable member 114. In some implementations, the barrier member 195 may be disposed on and/or coupled to an outer surface of the inflatable member 114. In some implementations, the barrier member 195 may be disposed within the inflatable member 114. For example, the barrier member 195 may be disposed on or coupled to an inner surface of the inflatable member 114 (e.g., opposite a surface of the inflatable member 114 that is intended to be pierced by the needle 130). In some implementations, the barrier member 195 may be disposed on or coupled to the elongated tube 112. In some implementations, the barrier member 195 may be disposed at a location between the elongated tube 112 and an inner surface of the inflatable member 114.

The barrier member 195 may have any suitable shape. In some implementations, the barrier member 195 may have a shape corresponding to a shape of an inner surface or an outer surface of the inflatable member 114 and/or a plane passing through the inflatable member 114. For example, the barrier member 195 may have an ovular profile, a circular profile, or a rectangular profile.

In some implementations, the barrier member 195 may be sufficiently resistant to piercing and/or tearing such that, if a needle (e.g., the needle 130) applies a greater force to the barrier member 195 than a magnetic attraction force applied by the external magnetic assembly 140 on the magnetic member 115 of the inflation assembly 110 (e.g., through the skin and trachea wall of the patient), the needle will urge the barrier member 195 toward the posterior tracheal wall of the patient and thus urge the magnetic member 115 away from the anterior tracheal wall of the patient, rather than the needle piercing the barrier member 195. Upon removal or reduction of the force of the needle on the barrier member 195 in the posterior direction, the magnetic member 115 may be urged again toward the anterior tracheal wall due to the magnetic attraction of the external magnetic assembly 140.

In some implementations, the barrier member 195 may be arranged relative to the magnetic member 115 such that, when the magnetic member 115 is urged toward the external magnetic assembly 140, the barrier member 195 is disposed opposite the magnetic member 115 from the external magnetic assembly 140 (e.g., between the magnetic member 115 and the posterior tracheal wall of the patient). In some implementations, the barrier member 195 may include one or more magnetic elements. The one or more magnetic elements may have a polarity relative to the magnetic member 115 and/or the external magnetic assembly 140 such that the barrier member 195 is repelled by the magnetic member 115 and/or the external magnetic assembly 140 such that the barrier member 195 will be urged toward the posterior tracheal wall and away from the anterior tracheal wall of the patient. The barrier member 195 may be coupled or disposed relative to the portion of the inflatable member 114 that is intended to be pierced such that, when the barrier member 195 is urged toward the posterior tracheal wall and away from the anterior tracheal wall of the patient (e.g., via magnetic interaction with the external magnetic assembly 140 and/or via the magnetic interaction of the external magnetic assembly 140 with the magnetic member 1150), the portion of the inflatable member 114 that is intended to be pierced is disposed near or adjacent to the anterior tracheal wall. Thus, the barrier member 195 may be disposed between an interior of the inflatable member 114 and the posterior tracheal wall such that, when a needle 130 is translated into the interior of the inflatable member 114, the barrier member 195 may prevent the needle 130 from being extended into contact with the posterior tracheal wall because further translation of the needle 130 may translate the needle 130 into contact with the barrier member 195.

In some implementations, the barrier member 195 may have increased echogenicity such that the barrier member 195 may be more easily visualized via ultrasound than other portions of the inflation assembly 110 (e.g., the inflatable member 114 and/or the interior of the inflatable member 114) and/or the surrounding portion of the patient. Due to the increased echogenicity, a user may be able to identify the location of the barrier member 195 via ultrasound imaging and discontinue translating the needle 130 prior to the needle reaching the barrier member 195 or prior to the needle passing the barrier member 195 such that the needle 130 may be prevented from advancing too far relative to the inflatable member 114 and/or the trachea of the patient and damaging the posterior tracheal wall of the patient.

In some implementations, the barrier member 195 may be formed of any suitable material with increased resistance to piercing by a needle used to pierce the tissue of a paitent (e.g., the needle 130) and/or increased echogenicity. For example, the barrier member 195 may be formed of a polymer or metallic composite. In some implementations, the barrier member 195 may include a thickened or strengthened portion of the sidewall of the inflatable member 114.

In some embodiments, the inflatable member 114 can be filled and/or inflated with a fluid (e.g., a liquid or a gaseous fluid) after being disposed in the upper trachea of the patient. For example, the inflatable member 114 can be filled and/or inflated with a fluid and/or contrast medium such that the inflatable member 114 defines an echogenic space detectable using ultrasound imaging. Inflating the inflatable member 114 can also increase the surface tension of the sidewall of the inflatable member such that the needle 130 and/or the guidewire 122 can more easily pierce the sidewall. Further, inflation of the inflatable member 114 can create a larger interior space within which the coupling member 124 can expand and/or be disposed. Inflation of the inflatable member 114 can also increase the target size of the inflatable member for visualization and targeting of the inflatable member 114 with the needle 130 and/or coupling member 124.

The tubular member 150 can have a first end 151 and a second end 153 opposite the first end 151. The tubular member 150 can define a lumen extending from the first end 151 to the second end 153. In some embodiments, the tubular member 150 can include an inflatable member 152 configured to extend from an outer surface of the tubular member 150 near the first end 151. The inflatable member 152 can be configured to seal against the inner surface of a trachea of a patient and/or stabilize the tubular member 150 within the trachea of the patient. The second end 153 of the tubular member 150 can be configured to be coupled to a source of ventilation such that when the first end 151 of the tubular member 150 is disposed within a trachea of a patient, the patient can be ventilated via the tubular member 150. In some embodiments, the tubular member 150 can be configured to be disposed within a trachea of a patient via translating the first end 151 of the tubular member 150 through a nasal or oral orifice of the patient. In some embodiments, the tubular member 150 can be an endotracheal tube. The tubular member 150 can be configured to receive at least a portion of the inflation assembly 110 within the lumen of the tubular member 150 such that the inflation assembly 110 can be translated relative to the first end 151 of the tubular member 150. In some embodiments, the tubular member 150 and the inflation assembly 110 are configured such that, when the inflation assembly 110 is disposed within the lumen of the tubular member 150 and translated relative to the tubular member 150, the tubular member 150 can continue ventilating the patient (e.g., providing air to the patient's lungs via the trachea of the patient). For example, in some embodiments, the outermost diameter or lateral extent of the inflation assembly 110 can be equal to or less than 50% of the inner diameter of the tubular member 150. In some embodiments, the inflation assembly 110 can be disposed in-line with the tubular member 150 such that the ventilator circuit is not broken and airway pressures through the trachea can be maintained.

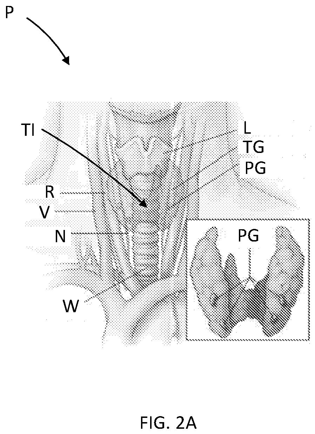

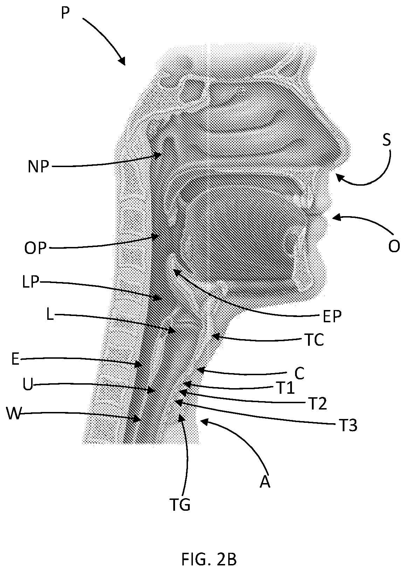

FIGS. 2A and 2B are schematic illustrations of an anterior and a cross-sectional side view of a portion of a patient P. As shown in FIGS. 2A and 2B, the patient P has an oral orifice O and a nasal orifice S. An end of a tubular member, such as the first end 151 of the tubular member 150, can be inserted through either the oral orifice O or the nasal orifice S and translated to a trachea W of the patient P. For example, the tubular member 150 can be inserted through the nasal orifice S and translated through the nasopharynx NP, the oropharynx O, passed the epiglottis EP, through the laryngopharynx LP avoiding the esophagus E, through the larynx L, passed the thyroid cartilage TC, through the cricoid ring C, and into the upper trachea U. The end of the tubular member can also be inserted through the oral orifice O and translated to the trachea W via the oropharynx OP.

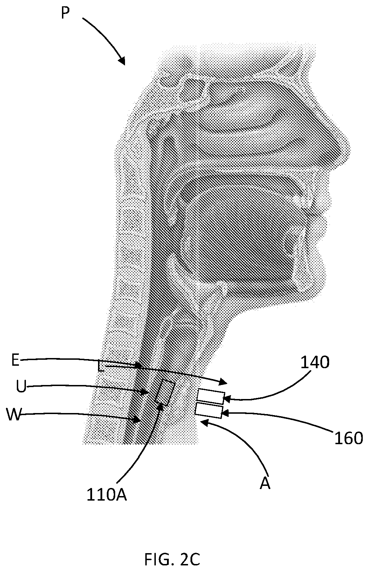

The ultrasound probe 160 can be any suitable ultrasound probe configured for visualization of the inflatable member 114 within the patient and any intervening patient structure between the skin of the patient and the inflatable member 114. For example, the ultrasound probe 160 may be used to visualize any intervening patient tissue or patient structures such as a wall of the trachea W, cartilage such as thyroid cartilage TC, blood vessels such as arteries R and/or veins V, nerves such as the laryngeal nerve N, the thyroid gland TG, a portion of the thyroid gland TG such as the thyroid isthmus TI, a parathyroid gland PG, and/or any other structures or tissue that may be disposed between the inflatable member 114 and the skin of the patient). The external magnetic assembly 140 can be any suitable external magnetic assembly configured to urge the magnetic member 115 of the inflation assembly 110 (e.g., via magnetic attraction) toward the external magnetic assembly through patient tissue (e.g., through the skin and trachea wall of the patient). As shown in FIG. 2C, the ultrasound probe 160 can be used to identify a location of a portion 110A of the inflation assembly 110 (e.g., a portion of the inflation assembly 110 including the inflatable member 114 and the magnetic member 115) within the patient P relative to other tissue or structures of the patient P. For example, as shown in FIG. 2C, with the inflation assembly 110 disposed within the patient (e.g., within the upper trachea U of the patient with the elongated tube 112 (not shown) extending from the portion 110A, through the larynx L, the laryngopharynx LP, the oropharynx OP, and out of the oral orifice O), the external magnetic assembly 140 can be coupled to an external surface of the patient (e.g., the anterior surface A of the skin of the patient's neck) such that the magnetic member 115 (not shown in FIG. 2C) of the portion 110A of the inflation assembly 110 is urged toward the external magnetic assembly 140 and the inflatable member 114 contacts a surface of a wall of the cavity (e.g., a surface of a wall of the upper trachea U). The inflatable member 114 and the external magnetic assembly 140 can be disposed on opposite sides of the intervening tissue and/or structures such that substantially no fluid (e.g., air) gaps are disposed between the inflatable member 114 and the external magnetic assembly 140. The surface of the wall of the cavity and the external surface can be disposed on opposite sides of at least one tissue surface of the patient. The inflatable member 114 can be visualized within the cavity. For example, the inflatable member 114 can be echogenic and visualized via the ultrasound probe 160. This technique, in which an echogenic member is urged against a surface of a wall of a body cavity, and the echogenic member and all tissue planes between the echogenic member and the external surface of the patient can be visualized by ultrasound, can be referred to as Coaptive Ultrasound (CU).

In some embodiments, the external magnetic assembly 140 can include a handle. In some embodiments, the external magnetic assembly 140 can include one magnetic element configured for magnetic interaction with the magnetic member 115. In some embodiments, the external magnetic assembly 140 can include any suitable number of magnetic elements (e.g., two magnetic elements) configured for magnetic interaction with the magnetic member 115. In some embodiments, as described above, the inflation assembly 110 can include a number of magnetic members 115 (e.g., two magnetic members), and the external magnetic assembly 140 can include a corresponding number of magnetic elements.

In some embodiments, the external magnetic assembly 140 and/or the magnetic member 115 can be formed of any suitable type of magnet. For example, the external magnetic assembly 140 and/or the magnetic member 115 can include a permanent magnet, such as a neodymium iron boron (NdFeB) magnet, a samarium cobalt (SmCo) magnet, an aluminum nickel cobalt (AlNiCo) magnet, a ceramic magnet, a ferrite magnet, and/or any other suitable rare earth magnet. In some embodiments, the external magnetic assembly 140 and/or the magnetic member 115 can include a temporary magnet. In some embodiments, the external magnetic assembly 140 and/or the magnetic member 115 can be an electromagnet, such as a solenoid. In some embodiments, the external magnetic assembly 140 and/or the magnetic member 115 can generate a magnetic field having an orientation (i.e., north (N) and south (S) poles). In other embodiments, the external magnetic assembly 140 and/or the magnetic member 115 can be formed of a ferromagnetic material that is not magnetized, i.e. does not generate its own magnetic field, but can be affected by an externally-applied magnetic field. For example, the external magnetic assembly 140 and/or the magnetic member 115 can be formed of iron or steel, and application of an external magnetic field can attract the iron toward the source of the field, applying a force to the external magnetic assembly 140 and/or the magnetic member 115.

In use, the tubular member 150 can be inserted through an orifice of a patient (e.g., a nose or mouth of a patient), through a cricoid ring of the patient, and into the upper trachea of the patient such that the first end 151 and the inflatable member 152 of the tubular member 150 are disposed within the upper trachea. For example, the inflatable member 152 of the tubular member 150 can be disposed between the second and third tracheal rings of the patient. The inflation assembly 110 can then be translated through the lumen of the tubular member 150 such that the first end 111 of the elongated member 112 of the inflation assembly 110 extends beyond the first end 151 of the tubular member 150. In some embodiments, a user can determine that the first end 111 of the elongated member 112 is extended a particular distance beyond the first end 151 of the tubular member 150 based on the known relative lengths of the tubular member 150 and the elongated member 112 and/or markings on at least one of the tubular member 150 or the elongated member 112.

The external magnetic assembly 140 can then be placed on the anterior neck of the patient such that the magnetic member 115 of the inflation assembly 110 is urged toward the external magnetic assembly 140 such that the first end 111 of the elongated member 112 is urged into contact with an anterior wall of the upper trachea. The tubular member 150 can be translated toward the cricoid ring relative to the first end 111 of the elongated member 112, which remains in position against the anterior wall of the upper trachea due to the magnetic attraction between the external magnetic assembly 140 and the magnetic member 115.

Fluid can then be delivered to the inflatable member 114 via the inflation lumen 116. As described above, the fluid can include a fluid and/or contrast medium such that the inflatable member 114 is detectable via imaging (e.g., ultrasound). The inflatable member 114 can then be visualized (e.g., using the ultrasound probe 160) such that the location of the inflatable member 114 can be identified. The external magnetic assembly 140 can then be moved along the skin of the anterior neck of the patient to urge the magnetic member 115 toward a desired tracheal puncture site. In some embodiments, the ultrasound probe 160 can be used to determine a tracheal puncture site between particular tracheal rings (e.g., between the first and second tracheal rings of the patient or between the second and the third tracheal rings of the patient).

While visualizing the location of the inflatable member 114 using the ultrasound probe 160, the guidewire assembly 120 can be inserted through the anterior neck of the patient and into the patient's trachea and coupled to the inflatable member 114. For example, the needle 130 can be inserted through the anterior neck and trachea of the patient and through a sidewall of the inflatable member 114 such that the first end 131 of the needle 130 (e.g., the tip) is disposed within the inflatable member 114. During insertion of the needle 130, the ultrasound probe 160 can be used to visualize the needle 130 and any intervening patient structure between the skin of the patient and the inflatable member 114. For example, the ultrasound probe 160 can be used to identify the thyroid isthmus and proximal blood vessels of the patient in real-time during insertion of the needle 130 such that the thyroid isthmus and proximal blood vessels can be avoided. Furthermore, the ultrasound probe 160 can be used to confirm that the first end 131 of the needle 130 is disposed within the inflatable member 114. Additionally or alternatively, echogenic fluid can be aspirated from the inflatable member 114 via the needle 130 (e.g., into a syringe barrel) to verify that the first end 131 of the needle 130 is disposed within the inflatable member 114.

With the first end 131 of the needle disposed within the inflatable member 114, the coupling member 124 and a portion of the guidewire 122 can be inserted through the lumen 135 of the needle 130 and translated (e.g., pushed) through the lumen 135. The coupling member 124 can then be translated out from the first end 131 of the needle 130 such that the coupling member 124 is disposed within the inflatable member 114. The needle 130 can then be withdrawn from the patient via translation of the needle 130 relative to the coupling member 124 and the guidewire 122, leaving the coupling member 124 within the inflatable member 114 and the guidewire 122 extending through a wall of the inflatable member 114. Additionally, the external magnetic assembly 140 can be removed from the patient such that the magnetic member 115 is no longer urged (e.g., via magnetic attraction) toward the anterior trachea wall. Additionally, the inflatable member 114 can be deflated.

With the guidewire assembly 120 extending through the anterior neck of the patient and coupled to the inflatable member 114, any suitable percutaneous tracheostomy procedure can be performed using the guidewire assembly 120 and the tract through the patient's anterior neck to the patient's trachea through which the guidewire assembly 120 is disposed. For example, the elongated member 112 can be translated through the tubular member 150 such that the first end 111 of the elongated member 112 moves toward the lower trachea and/or the lungs of the patient. Thus, the coupling member 124 and the guidewire 122 of the guidewire assembly 120 are translated toward the lower trachea and/or the lungs of the patient. External dilatation can then be performed via, for example, the Ciaglia technique or the Griggs technique. In some embodiments, the guidewire 122 will need to be advanced through the puncture site in the patient's neck as the elongated member 112 is translated to prevent the guidewire assembly 120 from decoupling with the inflatable member 114.

As another example, a translaryngeal tracheostomy (or Fantoni technique) can be performed using the guidewire assembly 120. For example, the tubular member 150 and the elongated member 112 can be translated such that the first end 111 of the elongated member 112 moves through an orifice (e.g., nasal or oral) of the patient. Thus, the coupling member 124 and the guidewire 122 of the guidewire assembly 120 are translated toward the cricoid ring and through the orifice of the patient. In some embodiments, the tubular member 150 can be withdrawn from the patient via the orifice of the patient prior to withdrawing the elongated member 112. A tracheostomy tube can then be threaded over the guidewire such that the tracheostomy tube can be translated through the patient's mouth, through the cricoid ring, into the upper trachea, and into engagement with the tract through the patient's trachea wall and anterior neck.

In some embodiments, to verify that the coupling member 124 of the guidewire is disposed within the trachea of the patient and engaged with the inflatable member 114, the elongated member 112 can be translated through the tubular member 150 such that the first end 111 of the elongated member 112 moves through an orifice (e.g., nasal or oral) of the patient. Thus, the coupling member 124 and the guidewire 122 of the guidewire assembly 120 are translated toward the cricoid ring and through or near the orifice of the patient. The engagement between the coupling member 124 and the inflatable member 114 can then be verified. After the verification, the guidewire 122 can be pulled through the tract in the anterior neck of the patient such that the coupling member 124 pulls the inflatable member 114 and the elongated member 112 into the upper trachea. Any suitable percutaneous tracheostomy can then be performed using the guidewire assembly 120.

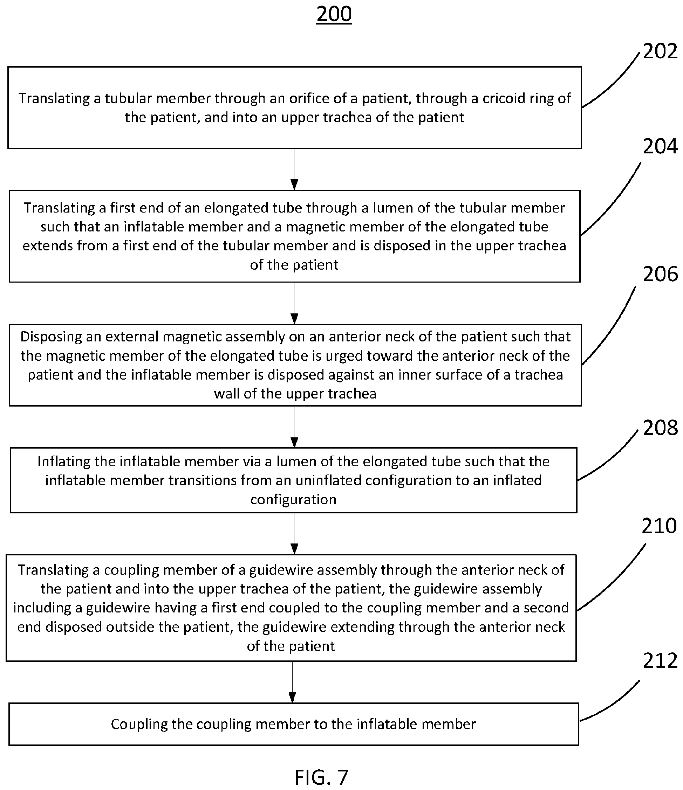

FIG. 7 is a flow chart of a method 200, according to an embodiment. The method 200 can be implemented using any of the systems or devices described herein, such as the system 100 described above. The method 200 includes translating 202 a tubular member through an orifice of a patient, through a cricoid ring of the patient, and into an upper trachea of the patient. A first end of an elongated tube can be translated 204 through a lumen of the tubular member such that an inflatable member and a magnetic member of the elongated tube extends from a first end of the tubular member and is disposed in the upper trachea of the patient. An external magnetic assembly can be disposed 206 on an anterior neck of the patient such that the magnetic member of the elongated tube is urged toward the anterior neck of the patient and the inflatable member is disposed against an inner surface of the upper trachea. The inflatable member can then be inflated 208 via a lumen of the elongated tube such that the inflatable member transitions from an uninflated configuration to an inflated configuration. A coupling member of a guidewire assembly can be translated 210 through the anterior neck of the patient and into the upper trachea of the patient. The guidewire assembly can include a guidewire having a first end coupled to the coupling member and a second end disposed outside the patient, the guidewire extending through the anterior neck of the patient. The coupling member can be coupled 212 to the inflatable member.

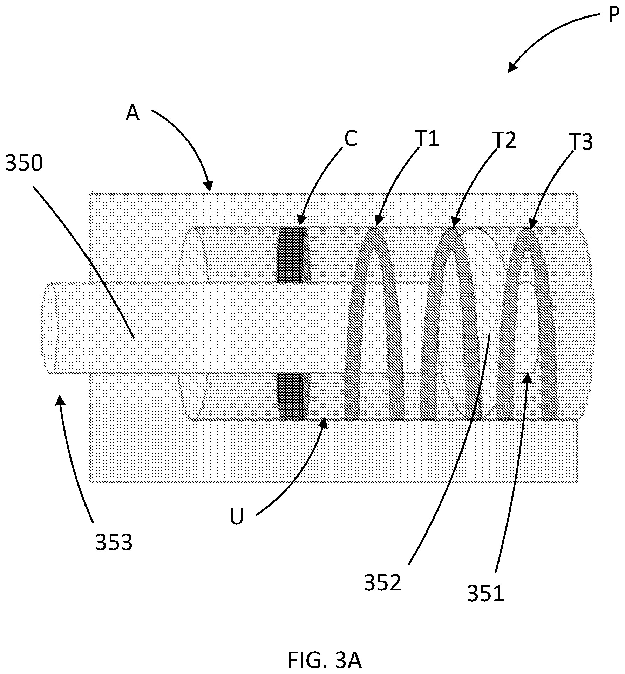

FIGS. 3A-3M are schematic illustrations of a system 300 in various stages of operation. The system 300 can be the same or similar in structure and/or function to any of the systems or devices described herein, such as the system 100 described above. For example, the system 300 includes an inflation assembly 310, a guidewire assembly 320, and a tubular member 350. The system 300 also includes an external magnetic assembly 340 and an ultrasound probe 360. The inflation assembly 310 can include an elongated tube 312, an inflatable member 314, and a magnetic member 315. The elongated tube 312 can have a first end 311 and a second end 313. In some embodiments, the elongated tube 312 can have a length sufficient to extend from at least an oral or nasal orifice of a patient to the trachea of the patient. The inflatable member 314 and the magnetic member 315 can be coupled to the elongated tube 312 near the first end 311 of the elongated tube 312. The inflation assembly 310 includes an inflation lumen 316 defined by the elongated tube 312 and in fluid communication with the inflatable member 314. The guidewire assembly 320 can include a guidewire 322 having a first end 321 and a second end 323 and a coupling member 324 disposed at the first end 321 of the guidewire 322. The tubular member 350 can have a first end 351 and a second end 353 opposite the first end 351. The tubular member 350 can define a lumen extending from the first end 351 to the second end 353 and can include an inflatable member 352 configured to transition from an uninflated configuration to an inflated configuration in which the inflatable member 352 extends from an outer surface of the tubular member 350 near the first end 351 and couples to the inner surface of the trachea wall of the patient.

As shown in FIG. 3A, the tubular member 350 can be inserted through an orifice (not shown) of a patient P (e.g., a nose or mouth of a patient), through a cricoid ring C of the patient P, and into the upper trachea U of the patient P such that the first end 351 and the inflatable member 352 of the tubular member 350 are disposed within the upper trachea U. The inflatable member 352 can be transitioned to the inflated configuration after the inflatable member 352 has been inserted through the cricoid ring C of the patient P such that the inflatable member 352 can secure the first end 351 within the upper trachea U and/or such that the first end 351 of the tubular member 350 can be stabilized (e.g., resistant to axial movement) relative to the inner surface of the trachea wall. For example, the inflatable member 352 of the tubular member 350 can be disposed between the second tracheal ring T2 and third tracheal ring T3 of the patient P or between the first tracheal ring T1 and the second tracheal ring T2 of the patient P. The inflatable member 352 can have any suitable shape. For example, the inflatable member 352 can have a circular profile in the inflated configuration such that a central axis of the first end 351 of the tubular member 350 can be coaxial with a central axis of the upper trachea U.

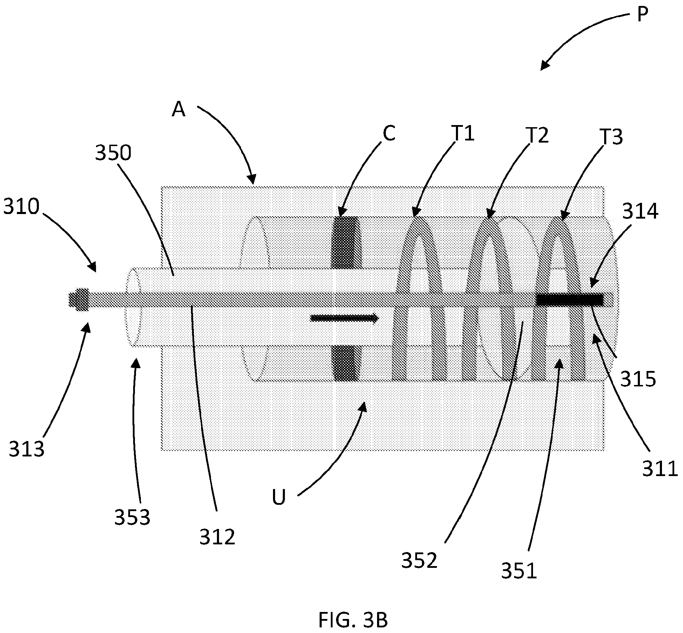

As shown in FIG. 3B, the inflation assembly 310 can then be translated through the lumen of the tubular member 350 such that the first end 311 of the elongated member 312 of the inflation assembly 310 extends beyond the first end 351 of the tubular member 350. In some embodiments, a user can determine that the first end 311 of the elongated member 312 is extended a particular distance beyond the first end 351 of the tubular member 350 based on the known relative lengths of the tubular member 350 and the elongated member 312 and/or markings on at least one of the tubular member 350 or the elongated member 312.

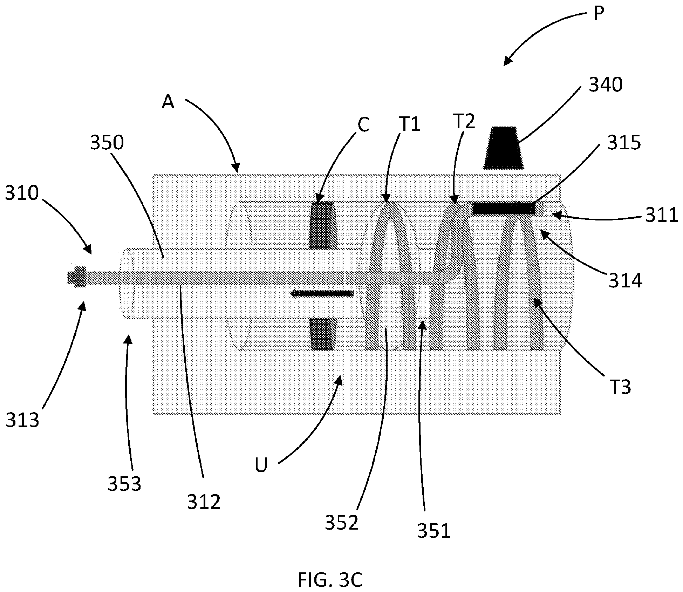

As shown in FIG. 3C, the external magnetic assembly 340 can then be placed on the anterior neck A of the patient such that the magnetic member 315 of the inflation assembly 310 is urged toward the external magnetic assembly 340 such that the first end 311 of the elongated member 312 is urged into contact with an anterior wall of the upper trachea U. The tubular member 350 can be translated toward the cricoid ring C relative to the first end 311 of the elongated member 312, which remains in position against the anterior wall of the upper trachea U due to the magnetic attraction between the external magnetic assembly 340 and the magnetic member 315. For example, in some implementations, the first end 351 of the tubular member 350 can be translated toward the cricoid ring C with the inflatable member 352 inflated by pulling the inflatable member 352 along the inner surface of the wall of the upper trachea U. In some implementations, the inflatable member 352 of the tubular member 350 can be partially or fully deflated prior to being translated toward the cricoid ring C, and then reinflated after being translated. In some implementations, the tubular member 350 can remain in its initial position relative to the cricoid ring C rather than translating the first end 351 of the tubular member 350 toward the cricoid ring C before proceeding (e.g., the inflatable member 352 can be initially inflated between the cricoid ring C and the first tracheal ring T1).

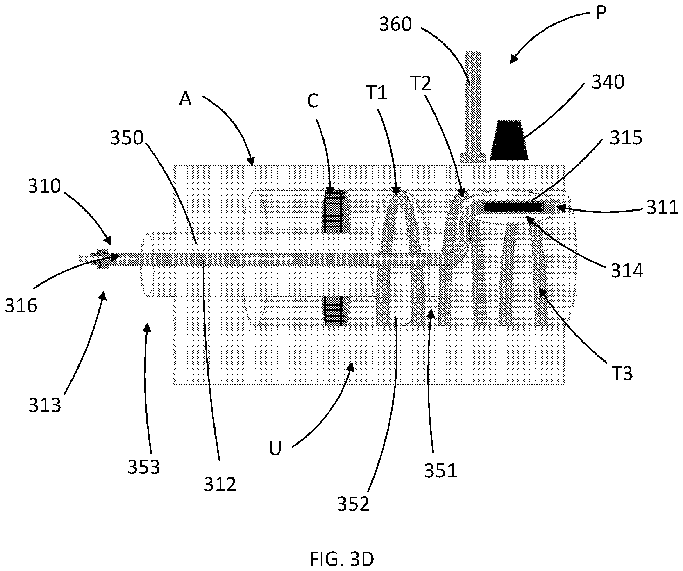

As shown in FIG. 3D, fluid can then be delivered to the inflatable member 314 via the inflation lumen 316 (e.g., via an inflation port coupled to the second end 313 of the elongated member 312). As described above, the fluid can include a fluid and/or contrast medium such that the inflatable member 314 is detectable via imaging (e.g., ultrasound). An ultrasound probe 360 can then be applied to the anterior neck A of the patient P such that the inflatable member 314 and any intervening tissue or other structure between the inflatable member and the surface of the anterior neck A can be visualized and the location of the inflatable member 314 can be identified. The external magnetic assembly 340 can then be moved along the skin of the anterior neck A of the patient to urge the magnetic member 315 toward a desired tracheal puncture site. In some embodiments, the ultrasound probe 360 can be used to determine a tracheal puncture site between particular tracheal rings (e.g., between the first and second tracheal rings T1, T2 of the patient P or between the second and the third tracheal rings T2, T3 of the patient P).

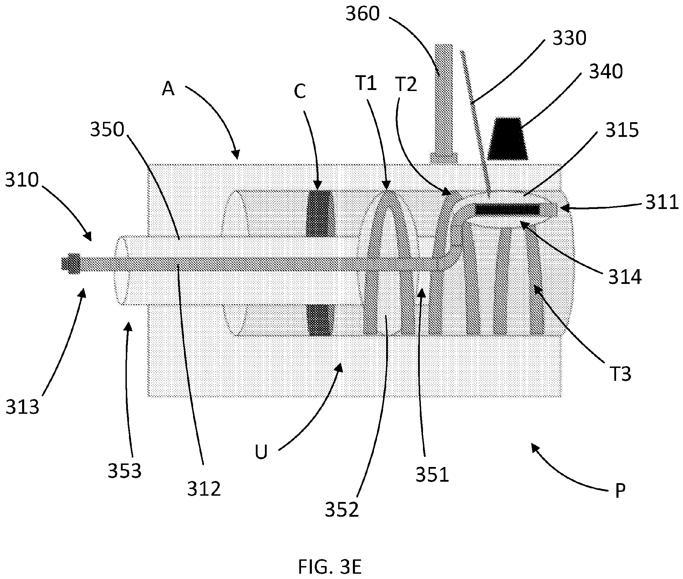

As shown in FIG. 3E, while visualizing the location of the inflatable member 314 using the ultrasound probe 360, the needle 330 can be inserted through the anterior neck A of the patient P and into the patient's upper trachea U. The needle 330 can be further translated such that the needle 330 is inserted through a sidewall of the inflatable member 314 such that the first end 331 of the needle 330 (e.g., the tip) is disposed within the inflatable member 314. During insertion of the needle 330, the ultrasound probe 360 can be used to visualize the needle 330 and any intervening patient structure between the skin of the patient and the inflatable member 314. For example, the ultrasound probe 360 can be used to identify the thyroid isthmus and proximal blood vessels of the patient in real-time during insertion of the needle 330 such that the thyroid isthmus and proximal blood vessels can be avoided. Furthermore, the ultrasound probe 360 can be used to confirm that the first end 331 of the needle 330 is disposed within the inflatable member 314. Additionally or alternatively, echogenic fluid can be aspirated from the inflatable member 314 via the needle 330 (e.g., into a syringe barrel) to verify that the first end 331 of the needle 330 is disposed within the inflatable member 314.

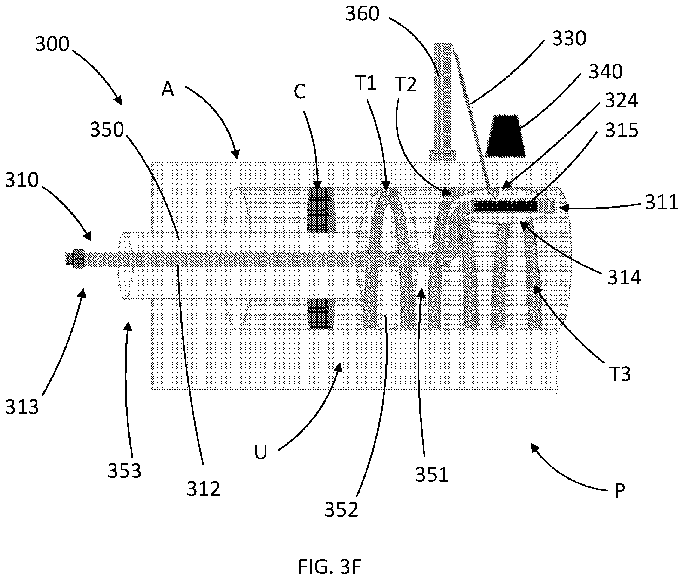

As shown in FIG. 3F, with the first end 331 of the needle disposed within the inflatable member 314, the coupling member 324 and a portion of the guidewire 322 can be inserted through the lumen 335 of the needle 330 and translated (e.g., pushed) through the lumen 335. The coupling member 324 can then be translated out from the first end 331 of the needle 330 such that the coupling member 324 is disposed within the inflatable member 314.

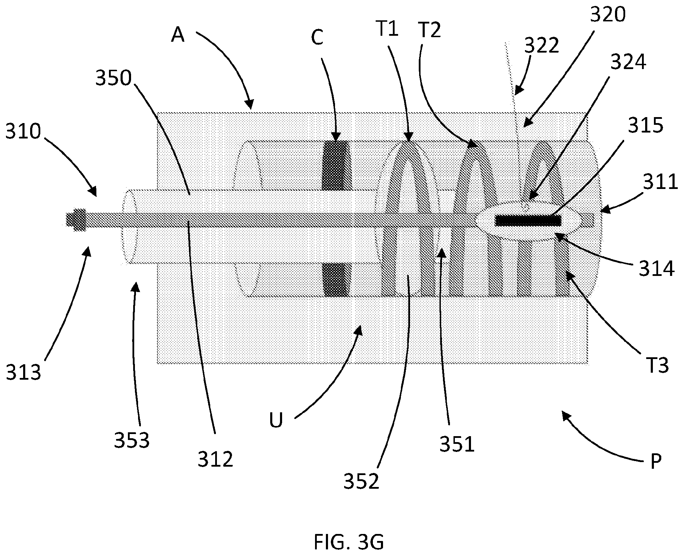

As shown in FIG. 3G, the needle 330 can then be withdrawn from the patient via translation of the needle 330 relative to the coupling member 324 and the guidewire 322, leaving the coupling member 324 within the inflatable member 314 and the guidewire 322 extending through a wall of the inflatable member 314. Additionally, the external magnetic assembly 340 can be removed from the patient such that the magnetic member 315 is no longer urged (e.g., via magnetic attraction) toward the anterior trachea wall. The inflatable member 314 can be deflated.

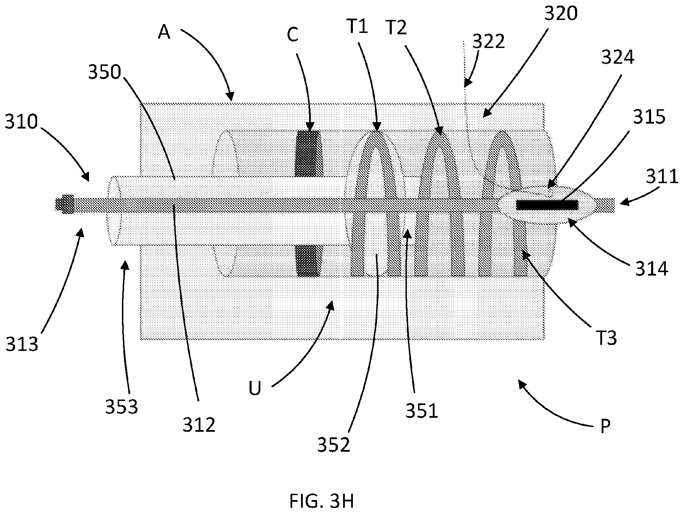

With the guidewire assembly 320 extending through the anterior neck of the patient and coupled to the inflatable member 314, any suitable percutaneous tracheostomy procedure can be performed using the guidewire assembly 320 and the tract through the patient's anterior neck to the patient's trachea through which the guidewire assembly 320 is disposed. For example, as shown in FIG. 3H, the elongated member 312 can be translated through the tubular member 350 such that the first end 311 of the elongated member 312 moves toward the lower trachea and/or the lungs of the patient. Thus, the coupling member 324 and the guidewire 322 of the guidewire assembly 320 are translated toward the lower trachea and/or the lungs of the patient. External dilatation can then be performed via, for example, the Ciaglia technique or the Griggs technique. In some embodiments, the guidewire 322 will need to be advanced through the puncture site in the patient's neck as the elongated member 312 is translated to prevent the guidewire assembly 320 from decoupling with the inflatable member 314. In some embodiments, the guidewire 322 may be pushed such that the inflatable member 314 is translated.

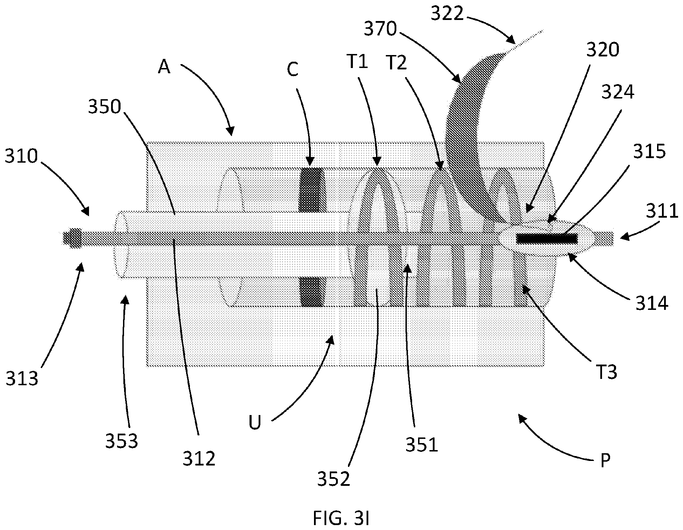

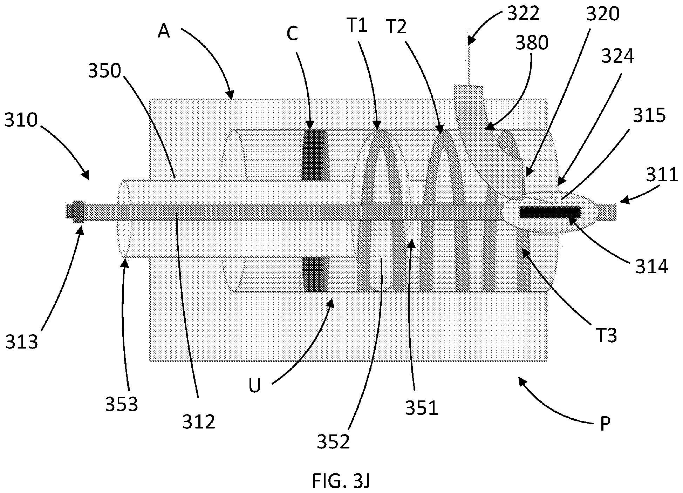

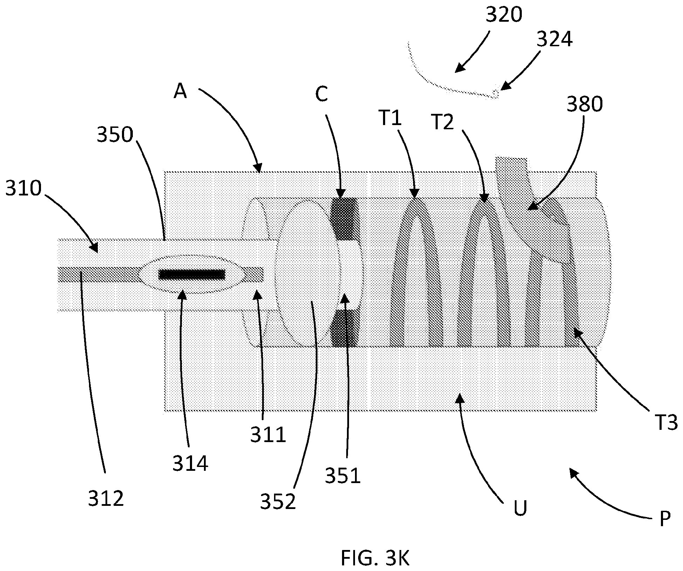

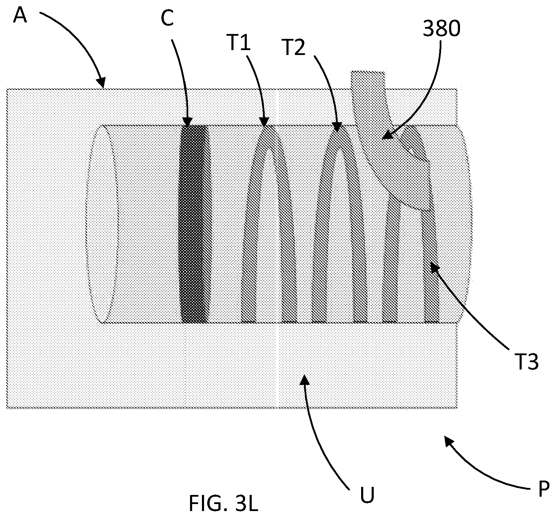

As an example of the Ciaglia technique, FIG. 3I shows that a dilator 370 can be advanced over the guidewire 322 to externally dilate the tract through the anterior neck A to the upper trachea U. As shown in FIG. 3J, after external dilation, a tracheostomy tube 380 can be advanced over the guidewire 322 such that a first end of the tracheostomy tube 380 is disposed within the upper trachea U and a second end of the tracheostomy tube 380 is disposed outside of the patient P (e.g., extending from the anterior neck A of the patient P). As shown in FIG. 3K, the guidewire assembly 320 can be uncoupled from the inflatable member 314. For example, a force sufficient to cause the coupling member 324 to decouple from the inflatable member 314 can be applied to the guidewire assembly 320 in a direction away from the patient P. The guidewire assembly 320 can then be translated through the tracheostomy tube 380 and removed from the patient P. The tracheostomy tube 380 can be coupled to a ventilator (not shown) such that the ventilator can push air into the lungs (not shown) of the patient P and draw air from the lungs of the patient P via the tracheostomy tube 380. After testing the ventilator and tracheostomy tube 380 combination to ensure that the ventilator is properly pushing air into the lungs of the patient P and drawing air from the lungs of the patient P via the tracheostomy tube 380, the inflation assembly 310 and tubular member 350 can be removed from the patient P. For example, the elongated member 312 can be translated proximally through the tubular member 350, and the elongated member 312 and the tubular member 350 can be sequentially or simultaneously removed from the patient P via proximal translation through an orifice of the patient P through which the tubular member 350 was inserted into the patient P. For example, the inflatable member 352 can be fully or partially deflated and the tubular member 350 can be proximally translated. As shown in FIG. 3L, the tracheostomy tube 380 can remain in place relative to the anterior neck A and upper trachea U of the patient, providing fluid flow access to the lungs of the patient.

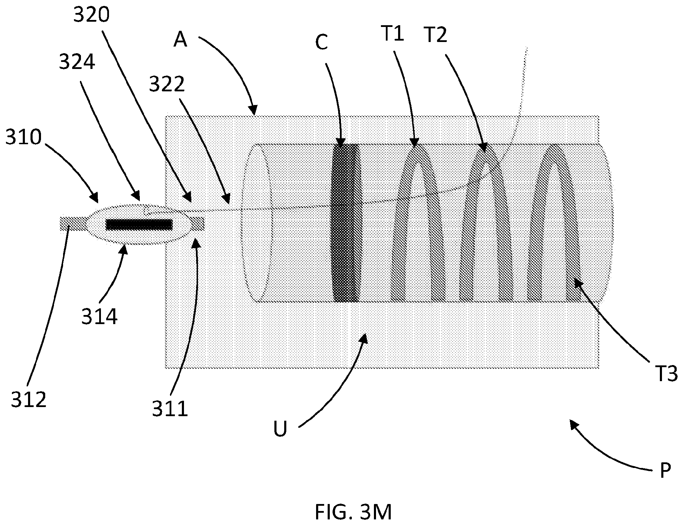

As another example, a translaryngeal tracheostomy or Fantoni technique can be performed using the guidewire assembly 320. For example, as shown in FIG. 3M, the tubular member 350 can be withdrawn from the patient P through an orifice (e.g., nasal or oral) of the patient P. The elongated member 312 can be translated such that the first end 311 of the elongated member 312 moves through the orifice of the patient P. Thus, the coupling member 324 and the guidewire 322 of the guidewire assembly 320 are translated through the cricoid ring C and out through the orifice of the patient P. A tracheostomy tube can then be threaded over the guidewire such that the tracheostomy tube can be translated through the patient's mouth, through the cricoid ring C, into the upper trachea U, and into engagement with the tract through the patient's trachea wall and anterior neck A.

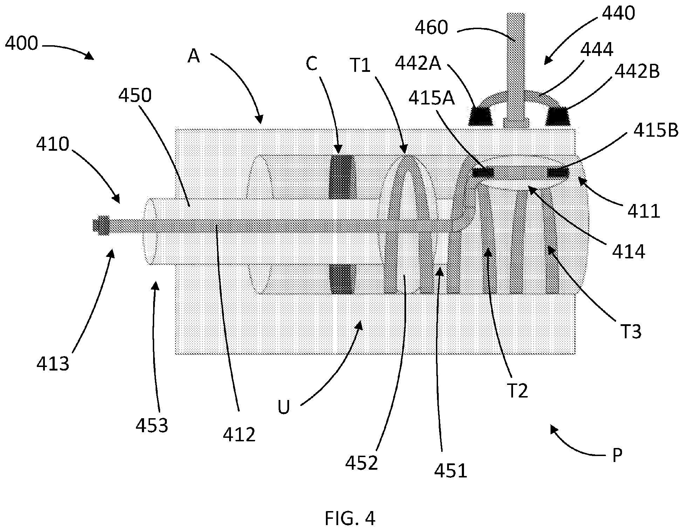

In some embodiments, the external magnetic assembly and the inflation assembly can include any suitable number of magnetic elements configured for magnetic interaction through tissue of the patient. For example, FIG. 4 is a schematic illustration of a system 400. The system 400 can be the same or similar in structure and/or function to any of the systems or devices described herein, such as the system 100 and/or the system 300 described above. For example, the system 400 includes an inflation assembly 410, a guidewire assembly 420, and a tubular member 450. The system 400 also includes an external magnetic assembly 440 and an ultrasound probe 460. The inflation assembly 410 can include an elongated tube 412 and an inflatable member 414. The elongated tube 412 can have a first end 411 and a second end 413. In some embodiments, the elongated tube 412 can have a length sufficient to extend from at least an oral or nasal orifice of a patient to the trachea of the patient. The inflatable member 414 and the magnetic member 415 can be coupled to the elongated tube 412 near the first end 411 of the elongated tube 412. The inflation assembly 410 includes an inflation lumen 416 defined by the elongated tube 412 and in fluid communication with the inflatable member 414. The inflation assembly 410 can also include a first magnetic member 415A and a second magnetic member 415B. The guidewire assembly 420 can include a guidewire 422 having a first end 421 and a second end 423 and a coupling member 424 disposed at the first end 421 of the guidewire 422. The tubular member 450 can have a first end 451 and a second end 453 opposite the first end 451. The tubular member 450 can define a lumen extending from the first end 451 to the second end 453 and can include an inflatable member 452 configured to extend from an outer surface of the tubular member 450 near the first end 451 and couple to the inner surface of the trachea wall of the patient.

The external magnetic assembly 440 can include a first magnetic element 442A, a second magnetic element 442B, and a handle 444. The first magnetic member 415A and the second magnetic member 415B of the inflation assembly 410 can be spaced along the elongated member 412 such that the first magnetic member 415A is configured for magnetic interaction with the first magnetic element 442A and the second magnetic member 415B is configured for magnetic interaction with the second magnetic element 442B. During use of the system 400, the ultrasound probe 460 can be positioned between the first magnetic element 442A and the second magnetic element 442B to visualize the inflatable member 414 of the inflation assembly 410. This embodiment also enables more control over the orientation of the first end 411 of the elongated tube 412, because its orientation will match the orientation of the external magnet assembly 440, i.e. of the first magnetic element 422A and the second magnetic element 422B.

In some embodiments, rather than having a plurality of magnetic members and/or elements, the inflation assembly 410 can include a disc-shaped magnetic member defining a through-hole and the external magnetic assembly 440 can include a disc-shaped magnetic element defining a through-hole. The ultrasound probe 460 can be inserted through the through-hole in the disc-shaped magnetic element of the external magnetic assembly 440 and into contact with the patient P.

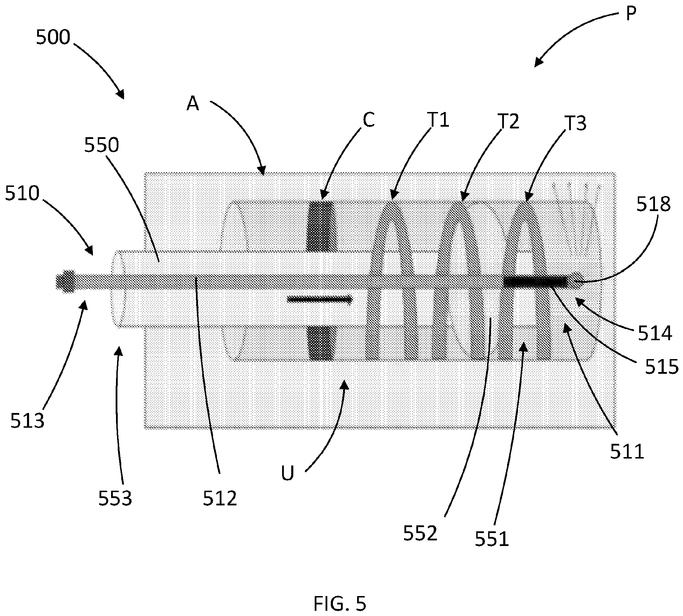

In some embodiments, as discussed above, a light source may be disposed on or near a first end of an elongated tube such that the location of the first end of the elongated tube within the patient can be identified by the location on the surface of the patient through which light is emitted. For example, FIG. 5 is a schematic illustration of a system 500. The system 500 can be the same or similar in structure and/or function to any of the systems or devices described herein, such as the system 100 and/or the system 300 described above. For example, the system 500 includes an inflation assembly 510 and a tubular member 550. The inflation assembly 510 can include an elongated tube 512 and an inflatable member 514. The elongated tube 512 can have a first end 511 and a second end 513. In some embodiments, the elongated tube 512 can have a length sufficient to extend from at least an oral or nasal orifice of a patient to the trachea of the patient. The inflatable member 514 and the magnetic member 515 can be coupled to the elongated tube 512 near the first end 511 of the elongated tube 512. The inflation assembly 510 includes an inflation lumen 516 defined by the elongated tube 512 and in fluid communication with the inflatable member 514. The tubular member 550 can have a first end 551 and a second end 553 opposite the first end 551. The tubular member 550 can define a lumen extending from the first end 551 to the second end 553 and can include an inflatable member 552 configured to extend from an outer surface of the tubular member 550 near the first end 551 and couple to the inner surface of the trachea wall of the patient.

As shown in Fig. 5, a light source 518 may be disposed on or near the first end 511 of the elongated tube 512. The light source may produce sufficient light such that light can emit from the light source, through the tracheal wall, to the surface of the anterior neck A and be visible to a user (e.g., a clinician). Thus, the user may be able to determine the location of the first end 511 of the elongated tube 512 based, at least in part, on the location of light emitting through the anterior neck A of a patient P. The light source 518 can be, for example, a light emitting diode (LED).

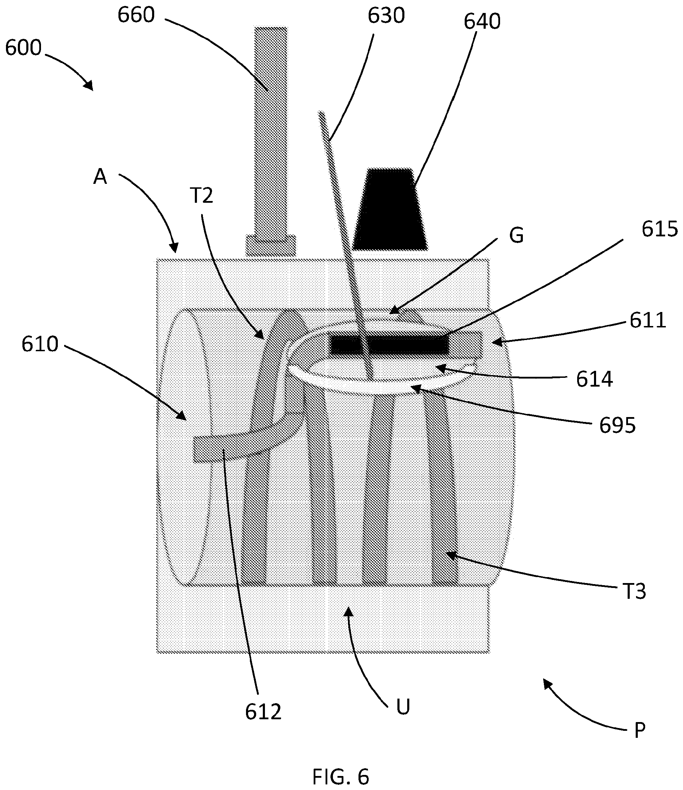

In some embodiments, as discussed above with respect to FIG. 1, an inflation assembly may include a barrier member to prevent a needle from puncturing a posterior tracheal wall of a patient. For example, FIG. 6 is a schematic illustration of a system 600. The system 600 can be the same or similar in structure and/or function to any of the systems or devices described herein, such as the system 100 and/or the system 300 described above. For example, the system 600 includes an inflation assembly 660. The system 600 also includes an external magnetic assembly 640, an ultrasound probe 660, and a needle 630. The inflation assembly 610 can include an elongated tube 612 and an inflatable member 614. The elongated tube 612 can have a first end 611 and a second end (not shown). In some embodiments, the elongated tube 612 can have a length sufficient to extend from at least an oral or nasal orifice of a patient to the trachea of the patient. The inflatable member 614 and the magnetic member 615 can be coupled to the elongated tube 612 near the first end 611 of the elongated tube 612. The inflation assembly 610 includes an inflation lumen defined by the elongated tube 612 and in fluid communication with the inflatable member 614.

As shown in FIG. 6, a barrier member 695 may be coupled to or form a portion of the sidewall of the inflatable member 614. The barrier member 695 may be the same or similar in structure and/or function as the barrier member 195 described above with respect to FIG. 1. For example, the barrier member 195 may have a shape that corresponds to a shape of the outer surface of the inflatable member 614. The barrier member 195 may be configured to be disposed between a portion of the inflatable member 614 intended to be pierced by the needle 630 and the posterior tracheal wall of the patient P. Furthermore, as shown in FIG. 6, the barrier member 695 may be sufficiently resistant to piercing and/or tearing such that, if the needle 630 applies a greater force to the barrier member 695 than a magnetic attraction force applied by the external magnetic assembly 640 on the magnetic member 615 of the inflation assembly 610 (e.g., through the anterior neck A and the trachea wall of the patient P), the needle 630 may urge the barrier member 695 toward the posterior tracheal wall of the patient P and thus urge the magnetic member 615 away from the anterior tracheal wall of the patient P, rather than the needle 630 piercing the barrier member 695 and/or passing through the barrier member 695. Thus, a gap G may exist between the outer surface of the inflatable member 614 and the inner surface of the anterior tracheal wall of the patient P when the needle 630 is urged against the barrier member 695 while the external magnetic assembly urges the magnetic member 615 toward the anterior neck A via magnetic attraction. Upon removal or reduction of the force of the needle 630 on the barrier member 695 in the posterior direction, the magnetic member 615 may be urged again toward the anterior neck A due to the magnetic attraction of the external magnetic assembly 640.

In some implementations, the barrier member 695 can be disposed inside the inflatable member 614 and coupled to an interior surface of a sidewall of the inflatable member 614. In some implementations, the barrier member 695 can be disposed outside of the inflatable member 614 and coupled to an exterior surface of a sidewall of the inflatable member 614. In some implementations, the inflatable member 614 can be partially formed of the barrier member 695. For example, the inflatable member 614 can include a first sidewall portion and a second sidewall portion opposite the first sidewall portion. The first sidewall portion can be configured to receive the needle 630 therethrough and the second sidewall portion can be configured to be more resistant to being pierced by the needle 630 than the first sidewall portion. For example, the second sidewall portion can have a greater thickness than the first sidewall portion and/or have a greater hardness than the first sidewall portion.

Additionally, in some implementations, the barrier member 695 may have increased echogenicity such that the barrier member 695 may be more easily visualized via ultrasound than other portions of the inflation assembly 610 (e.g., the inflatable member 614 and/or the interior of the inflatable member 614) and/or the surrounding portion of the patient P. Due to the increased echogenicity, a user may be able to identify the location of the barrier member 695 via ultrasound imaging and discontinue translating the needle 630 prior to the needle reaching the barrier member 195 or prior to the needle passing the barrier member 695 such that the needle 630 may be prevented from advancing too far relative to the inflatable member 614 and/or the trachea of the patient and damaging the posterior tracheal wall of the patient.

In some implementations, the barrier member 695 may be formed of any suitable material with increased resistance to piercing by a needle used to pierce the tissue of a patient (e.g., the needle 630) and/or increased echogenicity. For example, the barrier member 695 may be formed of a polymer or metallic composite. In some implementations, the barrier member 695 may include a thickened or strengthened portion of the sidewall of the inflatable member 614. The barrier member 695 may have an increased hardness (e.g., scratch hardness and/or indentation hardness) relative to the hardness of the inflatable member 614 or remainder of the inflatable member 614.

While various embodiments have been described above, it should be understood that they have been presented by way of example only, and not limitation. Where methods described above indicate certain events occurring in certain order, the ordering of certain events may be modified. Additionally, certain of the events may be performed concurrently in a parallel process when possible, as well as performed sequentially as described above.

Where schematics and/or embodiments described above indicate certain components arranged in certain orientations or positions, the arrangement of components may be modified. While the embodiments have been particularly shown and described, it will be understood that various changes in form and details may be made. Any portion of the apparatus and/or methods described herein may be combined in any combination, except mutually exclusive combinations. The embodiments described herein can include various combinations and/or sub-combinations of the functions, components, and/or features of the different embodiments described. The scope of the present invention is defined by the scope of the appended claims.

## Claims

1. A system (100) for performing a percutaneous tracheostomy, comprising:
an inflation assembly (110) including an elongated tube (112), an inflatable member (114), and a magnetic member (115), the elongated tube having a first end (111) , a second end (113), and defining a first lumen (116), the inflatable member coupled to the first end of the elongated tube, the inflatable member fluidically coupled to the first lumen such that the inflatable member can receive fluid via the first lumen, the magnetic member coupled to the first end of the elongated tube such that movement of the magnetic member can cause corresponding movement of the first end of the elongated tube;
a guidewire assembly (120) including a guidewire (122) having a first end (121) and a second end (123), the first end of the guidewire including a coupling member (124), the coupling member configured to couple to the inflatable member such that translation of the elongated tube translates the guidewire assembly; and
a tubular member (150) defining a second lumen, the second lumen configured to receive the inflation assembly (110) such that the inflation assembly is translatable within the second lumen.

2. The system of claim 1, wherein the inflatable member is a first inflatable member, the tubular member including a second inflatable member (152) configured to transition between an uninflated and an inflated configuration, the second inflatable member configured to extend from an outer surface of the tubular member in the inflated configuration.

3. The system of claim 2, wherein the second inflatable member (152) is configured to seal against an inner surface of a trachea in the inflated configuration.

4. The system of claim 1, wherein the tubular member is configured such that a patient can be ventilated via the tubular member when the inflation assembly is disposed within and translated relative to the second lumen.

5. The system of claim 1, wherein an outermost diameter of the inflation assembly is equal to or less than 50% of an inner diameter of the tubular member.

6. The system of claim 1, wherein the inflation assembly includes a barrier member (195) coupled to the inflatable member and configured to be more resistant to being pierced than a sidewall of the inflatable member.

7. The system of claim 6, wherein the barrier member has a first hardness and the sidewall of the inflatable member has a second hardness, the first hardness being greater than the second hardness.

8. The system of claim 6, wherein a portion of the sidewall of the inflatable member has a first thickness and the barrier member has a second thickness, the second thickness being greater than the first thickness.

9. The system of claim 1, wherein the inflatable member includes a first sidewall portion and a second sidewall portion opposite the first sidewall portion, the first sidewall portion configured to receive a needle therethrough and the second sidewall portion configured to be more resistant to being pierced by the needle than the first sidewall portion.

10. The system of claim 1, wherein the inflation assembly includes a barrier member disposed on or coupled to the elongated tube, the barrier member is more resistant to puncturing or tearing than the inflatable member or a portion of the inflatable member.

11. The system of claim 1, further comprising a needle (130) defining a third lumen (135) , the third lumen configured to receive the guidewire assembly.

12. The system of claim 1, further comprising a fluid configured to be disposed within the inflatable member via the first lumen of the elongated tube such that a location of the inflatable member can be visualized via ultrasound.

13. The system of claim 1, wherein the coupling member includes a shape memory material.

14. The system of claim 1, wherein the coupling member is configured to transition between a first configuration for insertion and a second configuration for retention or coupling, the coupling member having a greater lateral extent relative to a central axis of the guidewire in the second configuration than in the first configuration such that the coupling member can expand to retain the guidewire relative to the inflatable member in the second configuration and such that the coupling member can fit inside a lumen of a needle in the first configuration, the coupling member being biased toward the second configuration such that, in the absence of external forces on the coupling member, the coupling member will assume the second configuration.

15. The system of claim 14, wherein the first configuration of the coupling member is elongated such that the coupling member is shaped as a straight wire, and the second configuration of the coupling member corresponds to an unbiased shape or configuration that is a pigtail shape.

## Patentansprüche

1. System (100) zur Durchführung einer perkutanen Tracheostomie, umfassend:
eine Aufblähanordnung (110), die ein langgestrecktes Rohr (112), ein aufblähbares Element (114) und ein magnetisches Element (115) aufweist, wobei das langgestreckte Rohr ein erstes Ende (111) und ein zweites Ende (113) aufweist und ein erstes Lumen (116) definiert, wobei das aufblähbare Element an das erste Ende des langgestreckten Rohrs gekoppelt ist, wobei das aufblähbare Element fluidisch an das erste Lumen gekoppelt ist, so dass das aufblähbare Element Fluid über das erste Lumen erhalten kann, wobei das magnetische Element an das erste Ende des langgestreckten Rohrs gekoppelt ist, so dass Bewegung des magnetischen Elements entsprechende Bewegung des ersten Endes des langgestreckten Rohrs bewirken kann;
eine Führungsdrahtanordnung (120), die einen Führungsdraht (122) mit einem ersten Ende (121) und einem zweiten Ende (123) enthält, wobei das erste Ende des Führungsdrahts ein Kopplungselement (124) enthält, wobei das Kopplungselement zum Koppeln an das aufblähbare Element gestaltet ist, so dass Verschiebung des langgestreckten Rohrs die Führungsdrahtanordnung verschiebt; und
ein rohrförmiges Element (150), das ein zweites Lumen definiert, wobei das zweite Lumen zum Aufnehmen der Aufblähanordnung (110) gestaltet ist, so dass die Aufblähanordnung innerhalb des zweiten Lumens verschiebbar ist.

2. System nach Anspruch 1, wobei das aufblähbare Element ein erstes aufblähbares Element ist, wobei das rohrförmige Element ein zweites aufblähbares Element (152) enthält, das dafür gestaltet ist, zwischen einer nicht aufgeblähten und einer aufgeblähten Konfiguration überzugehen, wobei das zweite aufblähbare Element dafür gestaltet ist, sich in der aufgeblähten Konfiguration von einer Außenfläche des rohrförmigen Elements zu erstrecken.

3. System nach Anspruch 2, wobei das zweite aufblähbare Element (152) dafür gestaltet ist, in der aufgeblähten Konfiguration gegen eine Innenfläche einer Trachea abzudichten.

4. System nach Anspruch 1, wobei das rohrförmige Element so gestaltet ist, dass ein Patient über das rohrförmige Element beatmet werden kann, wenn die Aufblähanordnung innerhalb des zweiten Lumens angeordnet und relativ dazu verschoben ist.

5. System nach Anspruch 1, wobei ein äußerster Durchmesser der Aufblähanordnung gleich oder kleiner als 50 % eines Innendurchmessers des rohrförmigen Elements ist.

6. System nach Anspruch 1, wobei die Aufblähanordnung ein Barriereelement (195) enthält, das an das aufblähbare Element gekoppelt ist und dafür gestaltet ist, widerstandsfähiger gegen Durchstechen zu sein als eine Seitenwand des aufblähbaren Elements.

7. System nach Anspruch 6, wobei das Barriereelement eine erste Härte aufweist und die Seitenwand des aufblähbaren Elements eine zweite Härte aufweist, wobei die erste Härte größer als die zweite Härte ist.

8. System nach Anspruch 6, wobei ein Abschnitt der Seitenwand des aufblähbaren Elements eine erste Dicke aufweist und das Barriereelement eine zweite Dicke aufweist, wobei die zweite Dicke größer als die erste Dicke ist.

9. System nach Anspruch 1, wobei das aufblähbare Element einen ersten Seitenwandabschnitt und einen zweiten Seitenwandabschnitt gegenüber dem ersten Seitenwandabschnitt aufweist, wobei der erste Seitenwandabschnitt dafür gestaltet ist, eine Nadel hindurchgehend aufzunehmen, und der zweite Seitenwandabschnitt dafür gestaltet ist, widerstandsfähiger gegenüber Durchstechen durch die Nadel als der erste Seitenwandabschnitt zu sein.

10. System nach Anspruch 1, wobei die Aufblähanordnung ein Barriereelement enthält, das an dem langgestreckten Rohr angeordnet oder daran gekoppelt ist, wobei das Barriereelement widerstandsfähiger gegen Durchstechen oder Reißen ist als das aufblähbare Element oder ein Abschnitt des aufblähbaren Elements.

11. System nach Anspruch 1, ferner umfassend eine Nadel (130), die ein drittes Lumen (135) definiert, wobei das dritte Lumen dafür gestaltet ist, die Führungsdrahtbaugruppe aufzunehmen.

12. System nach Anspruch 1, ferner umfassend ein Fluid, das dafür gestaltet ist, über das erste Lumen des langgestreckten Rohrs in dem aufblähbaren Element angeordnet zu werden, so dass eine Position des aufblähbaren Elements über Ultraschall visualisiert werden kann.

13. System nach Anspruch 1, wobei das Kopplungselement ein Formgedächtnismaterial enthält.

14. System nach Anspruch 1, wobei das Kopplungselement dafür gestaltet ist, zwischen einer ersten Konfiguration zum Einführen und einer zweiten Konfiguration zum Halten oder Koppeln überzugehen, wobei das Kopplungselement eine größere laterale Ausdehnung relativ zu einer Mittelachse des Führungsdrahts in der zweiten Konfiguration als in der ersten Konfiguration aufweist, so dass sich das Kopplungselement ausdehnen kann, um den Führungsdraht relativ zu dem aufblähbaren Element in der zweiten Konfiguration zu halten, und so dass das Kopplungselement in ein Lumen einer Nadel in der ersten Konfiguration passen kann, wobei das Kopplungselement in Richtung zu der zweiten Konfiguration vorgespannt ist, so dass, bei Abwesenheit von äußeren Kräften auf das Kopplungselement, das Kopplungselement die zweite Konfiguration annehmen wird.

15. System nach Anspruch 14, wobei die erste Konfiguration des Kopplungselements langgestreckt ist, so dass das Kopplungselement als ein gerader Draht geformt ist, und die zweite Konfiguration des Kopplungselements einer ungespannten Form oder Konfiguration entspricht, die eine Spiralform ist.

## Revendications

1. Système (100) de réalisation d'une trachéostomie percutanée comprenant :
un ensemble de gonflage (110) comprenant un tube allongé (112), un élément gonflable (114), et un élément magnétique (115), le tube allongé ayant une première extrémité (111), une seconde extrémité (113), et définissant une première lumière (116), l'élément gonflable étant couplé à la première extrémité du tube allongé, l'élément gonflable étant couplé fluidiquement à la première lumière de sorte que l'élément gonflable puisse recevoir un fluide par l'intermédiaire de la première lumière, l'élément magnétique étant couplé à la première extrémité du tube allongé de sorte que le mouvement de l'élément magnétique puisse provoquer un mouvement correspondant de la première extrémité du tube allongé ;
un ensemble fil-guide (120) comprenant un fil-guide (122) ayant une première extrémité (121) et une seconde extrémité (123), la première extrémité du fil-guide comprenant un élément de couplage (124), l'élément de couplage étant configuré pour être couplé à l'élément gonflable de sorte que la translation du tube allongé entraîne la translation de l'ensemble fil-guide ; et
un élément tubulaire (150) définissant une deuxième lumière, la deuxième lumière étant configurée pour recevoir l'ensemble de gonflage (110) de sorte que l'ensemble de gonflage peut être translaté à l'intérieur de la deuxième lumière.

2. Système selon la revendication 1, l'élément gonflable étant un premier élément gonflable, l'élément tubulaire comprenant un second élément gonflable (152) configuré pour effectuer une transition entre une configuration non gonflée et une configuration gonflée, le second élément gonflable étant configuré pour s'étendre à partir d'une surface extérieure de l'élément tubulaire dans la configuration gonflée.

3. Système selon la revendication 2, le second élément gonflable (152) étant configuré pour assurer l'étanchéité contre une surface intérieure d'une trachée dans la configuration gonflée.

4. Système selon la revendication 1, l'élément tubulaire étant configuré de telle sorte qu'un patient peut être ventilé par l'intermédiaire de l'élément tubulaire lorsque l'ensemble de gonflage est disposé à l'intérieur de la deuxième lumière et translaté par rapport à celle-ci.

5. Système selon la revendication 1, un diamètre extérieur de l'ensemble de gonflage étant égal ou inférieur à 50 % d'un diamètre intérieur de l'élément tubulaire.

6. Système selon la revendication 1, l'ensemble de gonflage comprenant un élément formant barrière (195) couplé à l'élément gonflable et configuré pour être plus résistant à la perforation qu'une paroi latérale de l'élément gonflable.

7. Système selon la revendication 6, l'élément formant barrière ayant une première dureté et la paroi latérale de l'élément gonflable ayant une seconde dureté, la première dureté étant supérieure à la seconde dureté.

8. Système selon la revendication 6, une partie de la paroi latérale de l'élément gonflable ayant une première épaisseur et l'élément formant barrière ayant une seconde épaisseur, la seconde épaisseur étant supérieure à la première épaisseur.

9. Système selon la revendication 1, l'élément gonflable comprenant une première partie de paroi latérale et une seconde partie de paroi latérale opposée à la première partie de paroi latérale, la première partie de paroi latérale étant configurée pour recevoir une aiguille à travers celle-ci et la seconde partie de paroi latérale étant configurée pour être plus résistante à la perforation par l'aiguille que la première partie de paroi latérale.

10. Système selon la revendication 1, l'ensemble de gonflage comprenant un élément formant barrière disposé sur ou couplé au tube allongé, l'élément formant barrière étant plus résistant à la perforation ou à la déchirure que l'élément gonflable ou une partie de l'élément gonflable.

11. Système selon la revendication 1, comprenant en outre une aiguille (130) définissant une troisième lumière (135), la troisième lumière étant configurée pour recevoir l'ensemble fil-guide.

12. Système selon la revendication 1, comprenant en outre un fluide configuré pour être disposé à l'intérieur de l'élément gonflable par l'intermédiaire de la première lumière du tube allongé de sorte qu'un emplacement de l'élément gonflable peut être visualisé par ultrasons.

13. Système selon la revendication 1, l'élément de couplage comprenant un matériau à mémoire de forme.

14. Système selon la revendication 1, l'élément de couplage étant configuré pour effectuer une transition entre une première configuration d'insertion et une seconde configuration de retenue ou de couplage, l'élément de couplage présentant une plus grande étendue latérale par rapport à un axe central du fil-guide dans la seconde configuration que dans la première configuration, de sorte que l'élément de couplage peut se dilater pour retenir le fil-guide par rapport à l'élément gonflable dans la seconde configuration et que l'élément de couplage peut s'insérer dans une lumière d'une aiguille dans la première configuration, l'élément de couplage étant sollicité vers la seconde configuration de sorte que, en l'absence d'efforts extérieurs sur l'élément de couplage, l'élément de couplage prendra la seconde configuration.

15. Système selon la revendication 14, la première configuration de l'élément de couplage étant allongée de telle sorte que l'élément de couplage a la forme d'un fil droit, et la seconde configuration de l'élément de couplage correspond à une forme ou une configuration non sollicitée qui est une forme en queue de cochon.
